# EUROPÄISCHE PATENTANMELDUNG

(11) **EP 2 364 715 A1**
(43) Veröffentlichungstag der Anmeldung: **14.09.2011**
(21) Anmeldenummer: 10197086.1
(22) Anmeldetag: 27.12.2010
(51) Int. Cl.: A61K 36/41

(54) **Rhodiolapräparat mit Astaxanthin und Vitaminen**

(30) Priorität: 28.12.2009 DE 102009060816
(71) Anmelder: Dr. Loges + Co. GmbH, 21423 Winsen, Luhe (DE)
(72) Erfinder: Dr. Biller, Andreas, 21423, Winsen (Luhe) (DE)
(74) Vertreter: Wenzel & Kalkoff

(57) **Zusammenfassung**

Eine pharmazeutische, medizinische, diätetische oder alimentäre Zusammensetzung basierend auf einer Kombination aus Rhodiola-Extrakt, Astaxanthin und einem Antioxidans, ausgewählt aus der Gruppe Vitamin A, Vitamin C und Vitamin E, fördert die Regeneration nach sportlicher Belastung, verbessert die Trainierbarkeit von Ausdauerleistungen und steigert die motorische und kognitive Leistungsfähigkeit nach körperlicher Erschöpfung. So belegte ein Test, bei dem die Leistungssteigerung im Ausdauertraining unter Einnahme bzw. ohne Einnahme des erfindungsgemäßen Präparats verglichen wurde, eine deutlich größere Leistungssteigerung bei der Verumgruppe.

## Beschreibung

Die vorliegende Erfindung betrifft eine pharmazeutische, medizinische, diätetische oder alimentäre Zusammensetzung.

Körperliche Belastung ruft Stressauswirkungen auf verschiedenen Ebenen aufgrund der kardiozirkulatorischen, biochemischen, endokrin- und zentralnevörs-regulativen sowie psychisch-kognitiven Anforderungen und der nachfolgenden Anpassungsreaktionen hervor. Bei wenig ausdauertrainierten Menschen tritt aufgrund des erhöhten Sauerstoffumsatzes bei entsprechender Belastung oxidativer Stress, der in der Pathophysiologie des Übertrainingssyndroms und vieler Krankheitsprozesse sowie bei der biologischen Alterung eine Rolle spielt, hinzu. Für die Leistungsfähigkeit bei körperlicher Belastung ist die Regeneration zwischen den einzelnen Belastungseinheiten von entscheidender Bedeutung. Diese Regeneration kann neben zahlreichen anderen Maßnahmen insbesondere auch durch die Ernährung unterstützt und positiv beeinflusst werden.

Es sind zahlreiche, unterschiedliche Präparate mit ganz unterschiedlichen Wirkstoffen bekannt, die über verschiedene, zum Teil nicht geklärte Mechanismen die Regeneration fördern und beschleunigen. So ist der Rhodiola rosea (Rosenwurz) mit den hauptsächlichen Wirkstoffen Salidroside und Rosavin (Phenylethanol und Phenylpropanoid) als adaptogen charakterisiert, d.h. als für den Organismus hilfreich, sich an Stresssituationen anzupassen bzw. einen positiven Effekt bei Stress-induzierten Krankheiten auszuüben. Rhodiola rosea soll allgemein die Widerstandskraft gegen unterschiedliche Formen von Stress erhöhen und durch zentralnervöse Stabilisierung der Erschöpfung entgegenwirken sowie Konzentration, geistige Frische und Gedächtnisfähigkeit in Stress-Situationen erhalten. Allerdings kommen die zu Rhodiola-Applikation im Zusammenhang mit sportlicher Beanspruchung durchgeführten Studien nicht zu einheitlichen Ergebnissen (De Bock et al, "Acute Rhodiola rosea intake can improve endurance exercise performance" Int J Sport Nutr Exerc Metab. 2004; 14(3):298-307). De Bock et al. untersuchten akute und chronische Applikationen und fanden nur eine Verbesserung im Ausdauerleistungsbereich, nicht jedoch in den Bereich Kraft, Schnelligkeit, Aufmerksamkeit und Reaktion. Die Wirkung auf die Dauerleistung wurde bei Ratten bestätigt (Abidov et al. 2004).

Eine weitere Substanz, die im Hinblick auf Verbesserung der Leistungsfähigkeit untersucht worden war, ist Astaxanthin. Astaxanthin gehört zu den Carotinoiden und weist eine sehr hohe antioxidative Kapazität auf. Es wird aus der Süßwasseralge Haematococcus pluvialis gewonnen. Verschiedene Versuche weisen darauf hin, dass sich durch Astaxanthin auch oxidativer Stress reduzieren lässt. Astaxanthin wurde im Tierversuch mit Mäusen in Hinblick auf seinen Einfluss auf den Lipidstoffwechsel im Muskel untersucht (AOI, W. et al.: "Astaxanthin improves muscle lipid metabolism in exercise via inhibitory effect of oxidative CPT I modification", Biochem. Biopyhs. Res. Commun. 366 (4), 2008, S. 892-897; ISBN: 0006-291X). Die Studie ergab, dass Astaxanthin den Lipidstoffwechsel der Muskeln bei körperlichem Training aufgrund seiner antioxidativen Wirkung verbessert. Im Humanversuch zeigte Astaxanthin keinen Effekt auf Kraft, Schmerz und CK-Blutwerte (Creatin-Kinase-Blutwerte) bei/nach hoher exzentrischer Kraftbelastung (Bloomer et al 2005).

Überraschenderweise wurde jetzt gefunden, dass eine Kombination aus Rhodiola-Extrakt, Astaxanthin und einem Antioxidans, ausgewählt aus der Gruppe Vitamin A, Vitamin C und Vitamin E die Regeneration nach sportlicher Belastung fördert, die Trainierbarkeit von Ausdauerleistungen wie auch von Kraftbelastungen verbessert und dabei gleichzeitig die motorische und kognitive Leistungsfähigkeit nach körperlicher Erschöpfung steigert. So belegte ein Test, bei dem die Leistungssteigerung im Ausdauertraining unter Einnahme (Verumgruppe) bzw. ohne Einnahme (Placebo-Gruppe) des erfindungsgemäßen Präparats verglichen wurde, eine deutlich größere Leistungssteigerung bei der Verumgruppe. Der Leistungszuwachs in der Placebo-Gruppe wurde u.a. durch eine leichte Sauerstoffschuld erkauft. In der Verum-Gruppe wurde hingegen nach der anstrengenden Trainingsperiode ein niedrigerer Laktatspiegel gemessen, was belegt, dass die erfindungsgemäße Zusammensetzung zu mehr aerober Leistung führt. Die erfindungsgemäße Zusammensetzung führt zu einer besseren Anpassung im submaximal-aeroben Bereich mit gleichzeitiger Laktat-Senkung.

Unerwartet wurde somit gefunden, dass die erfindungsgemäße Zusammensetzung eine Langzeitwirksamkeit bezüglich der Regenerationsfähigkeit im Training bewirkt. Ferner konnte gezeigt werden, dass ein sog. "Könnerzustand" erreicht wird. So war in der Verum-Gruppe die Theta-Frequenz (Aufmerksamkeitssteuerung) erkennbar günstiger. Zahlreiche Gruppenunterschiede im EEG-Muster waren in der Erholungsphase nachweisbar.

Das Erreichen eines "Könnerzustands" oder "Expertenzustands" bedeutet, dass zwar keine Änderung in der kognitiven Leistungsfähigkeit eintritt, aber weniger Aufwand für die Erbringung der Leistung erforderlich ist, was eine schnellere Regeneration ermöglicht. Ein höherer Aufmerksamkeitslevel führt eine Ökonomisierung der corticalen Vorgänge herbei. Dies ist vergleichbar mit dem Erreichen eines höheren Könnenszustandes an sich, denn es ist nachgewiesen, dass "Experten" gleiche Aufgaben mit geringerer Aktivierung und geringerer Aktivitätsausbreitung im Gehirn erreichen, also weniger Ressourcen für die gleiche Leistung in Anspruch nehmen. Dies ist insbesondere relevant für alle Sportarten, in denen es neben Ausdauer auch auf kognitive Fähigkeiten ankommt, wie z.B. beim Fußball, Tennis oder Handball. Hier kann die Reaktionsfähigkeit und die Aufmerksamkeit über einen längeren Zeitraum auf höherem Niveau gehalten werden als dies ohne Einnahme der erfindungsgemäßen Zusammensetzung der Fall wäre.

Die erfindungsgemäße Zusammensetzung führt somit in überraschender Weise zum Erreichen der Kombination zweier vorteilhafter Wirkungen, nämlich einer verbesserten Regenerationsfähigkeit in Verbindung mit einer Steigerung der Konzentrationsfähigkeit und Aufmerksamkeit.

Vorzugsweise ist der Rhodiola-Extrakt ein hydro-ethanolischer Rhodiola rosea-Extrakt. Rhodiola rosea beeinflusst Gesundheit und geistige Leistungsfähigkeit positiv, indem es die Ausschüttung der Botenstoffe im Gehirn stimuliert und für das richtige Verhältnis der Botenstoffe zueinander sorgt. Die adaptogene Wirkung von Rhodiola rosea zeigt sich in einer gesteigerten geistigen Wachheit, Aufmerksamkeit und Entscheidungsfähigkeit. Darüber hinaus weist es antioxidative Inhaltsstoffe auf, die freie Radikale unschädlich machen und somit die Zerstörung der Umhüllungen der Zellen und daraus bedingte Entzündungen verhindern können. Ein wässrig-ethanolischer Extrakt mit 60% Ethanol-Anteil hat sich dabei als besonders vorteilhaft herausgestellt.

Besonders geeignet als Antioxidans ist Vitamin E. Vitamin E ist ein fettlösliches Antioxidans, das die ungesättigten Fettsäuren im Körper, die u.a. wesentlicher Baustein der Zell- und Organmembranen sind, schützt, indem es freie Radikale, die die ungesättigten Fettsäuren oxidieren würden, fängt. Die Wirkung des Vitamin E wird durch die hohe zellschützende Potenz des Astaxanthins ergänzt und unterstützt.

Eine besonders gute Wirkung lässt sich erzielen, wenn das Präparat des Weiteren mindestens einen Mineralstoff, ausgewählt aus der Gruppe Calcium, Kalium, Magnesium, Natrium, Chlor, Eisen, Selen und Zink, enthält. So sind beispielsweise Magnesium und Kalium auf vielfältige Weise in die Stoffwechselvorgänge des Körpers involviert. Körperliche Belastung bewirkt erhöhte Mineralstoffumsätze. Ein durch körperliche Belastung bedingter Mangel an diesen Mineralstoffen würde sich negativ auf die Belastungsfähigkeit auswirken, so dass eine Substitution durch Beifügung dieser Mineralstoffe zum Präparat einem etwaigen Mangel entgegenwirkt und die Leistungsfähigkeit erhält. So wird durch Zugabe von Magnesium der durch häufiges Schwitzen entstehende Mehrbedarf bei Sportlern an diesem Mineralstoff ergänzt. Vorteilhaft enthält das Präparat neben Magnesium insbesondere auch Kalium, da dies die Aufnahme von Magnesium im Körper noch verbessert.

In einer besonders bevorzugten Zusammensetzung enthält damit die Wirkstoffzusammensetzung zwischen 10 und 75 Gew.-%, vorzugsweise zwischen 20 und 45 Gew.-%, besonders bevorzugt zwischen 30 und 45 Gew.-% Rhodiola-Extrakt, zwischen 2 und 30 Gew.-%, vorzugsweise zwischen 6 und 25 Gew.-%, besonders bevorzugt zwischen 9 und 21 Gew.-% Antioxidans, zwischen 0,1 und 15 Gew.-%, vorzugsweise zwischen 0,2 und 6 Gew.-%, besonders bevorzugt zwischen 0,3 und 3 Gew.-% Astaxanthin und zwischen 0 und 87,9 Gew.-%, vorzugsweise zwischen 25 und 75 Gew.-%, besonders bevorzugt zwischen 31 und 60,7 Gew.-% mindestens eines Mineralstoffes, ausgewählt aus der Gruppe Calcium, Kalium, Magnesium, Natrium, Chlor, Eisen, Selen und Zink.. In einer solchen Zusammensetzung ist das Zusammenspiel von Rhodiola-Extrakt, Astaxanthin und Antioxidans besonders gut, die radikalfangende Wirkung ist in besonders hohem Maße verstärkt, und die Regenerationsdauer eines Organismus kann vorteilhaft verringert werden.

Weitere für den Organismus, gerade bei Sporttreibenden wichtige Stoffe sind zum Beispiel Lecithin, L-Carnitin, oder Vitamine des B-Komplexes, essenzielle Aminosäuren und/oder essenzielle Fettsäuren, so dass ein Präparat besonders bevorzugt ist, das zusätzlich von diesen Stoffen einen oder mehrere enthält.

Daher enthält die Wirkstoffzusammensetzung in einer besonders vorteilhaften Zusammensetzung zwischen 10 und 75 Gew.-%, vorzugsweise zwischen 20 und 45 Gew.-%, besonders bevorzugt zwischen 30 und 45 Gew.-% Rhodiola-Extrakt, zwischen 2 und 30 Gew.-%, vorzugsweise zwischen 6 und 25 Gew.-%, besonders bevorzugt zwischen 9 und 21 Gew.-% Antioxidans, zwischen 0,1 und 15 Gew.-%, vorzugsweise zwischen 0,2 und 6 Gew.-%, besonders bevorzugt zwischen 0,3 und 3 Gew.-% Astaxanthin, zwischen 5 und 90 Gew.-%, vorzugsweise zwischen 10 und 75 Gew.-% und besonders bevorzugt zwischen 25 und 45 Gew.-% mindestens einer Verbindung ausgewählt aus der Gruppe Lecithin, L-Carnitin, Vitamine des B-Komplexes, essenzielle Aminosäuren und essenzielle Fettsäuren und zwischen 5 und 90 Gew.-%, vorzugsweise zwischen 10 und 75 Gew.-%, besonders bevorzugt zwischen 25 und 45 Gew.-% mindestens eines Mineralstoffes, ausgewählt aus der Gruppe Calcium, Kalium, Magnesium, Natrium, Chlor, Eisen, Selen und Zink.

Als Darreichungsform des erfindungsgemäßen Präparates ist insbesondere die Weichgelatinekapsel bevorzugt, weil diese u.a. einfach und für den Anwender angenehm einzunehmen ist. Weitere bevorzugte Darreichungsformen sind Hartgelatinekapseln, Pulver, Tabletten, insbesondere Brausetabletten, Lutschtabletten, Kautabletten oder Dragees, Lösungen, Säfte oder Sirupe.

Die erfindungsgemäße Zusammensetzung kann neben den genannten Wirkstoffen auch sogenannte Hilfsstoffe umfassen, die der Fachmann je nach Anwendung frei wählen kann.

Dabei sind als Träger bzw. Füllstoffe Glycerin, Mono-, Di- und Triglyceride, Stärkederivate sowie Cellulosederivate und insbesondere Pflanzenöle wie Rapsöl oder Kokosöl besonders geeignet.

Bevorzugte Bindemittel sind Polyvinylpyrrolidone sowie Polyethylenglykole oder Gelatine.

Als Trenn- bzw. Gleitmittel kommen vorteilhafter Weise Siliziumdioxid, Magnesiumstearat, Lecithin, Cellulose und/oder Carboxymethylstärke zum Einsatz.

Für einen besonders angenehmen Geschmack kann die Zusammensetzung zusätzlich Süßungsmittel und/oder Aromen umfassen, wobei Maltodextrin, Fructose, Glucose und Sorbit vorzugsweise zum Einsatz kommen.

Um den optischen Eindruck ansprechend zu gestalten, können der Zusammensetzung darüber hinaus Farbstoffe zugesetzt sein, vorzugsweise Titandioxid (E 171) sowie Eisenoxid rot und/oder schwarz (E 172).

Die erfindungsgemäße Zusammensetzung ist besonders geeignet zur Förderung der Regeneration nach sportlicher Belastung sowie zur Verbesserung der Trainierbarkeit von Ausdauerbelastungen und zur Steigerung der motorischen und kognitiven Leistungsfähigkeit nach körperlicher Belastung bzw. Erschöpfung.

Dabei haben sich als vorteilhaft folgende Tagesdosen herausgestellt: Zwischen 200 und 300 mg Rhodiola-Extrakt, zwischen 2 und 7 mg Astaxanthin und zwischen 80 und 150 mg Antioxidans, ausgewählt aus der Gruppe Vitamin A, Vitamin C und Vitamin E.

Beispielrezeptur:

| | |
|---|---|
| **Kapselinhalt:** | **Gehalt [mg]** |
| Magnesiumoxid, schwer | 110,539 |
| Tri-Kaliumcitrat | 92,192 |
| D-α-Tocopherol, 1000 IE/g | 39,74 |
| Rhodiola Rosea Extrakt | 66,666 |
| Astaxanthin Komplex 7% | 19,04 |
| **Hilfsstoff:** | |
| Rapsöl, raff., Kokosöl, raff., Lecithin (Raps) | 231,954 |
| **Summe Kapselinhalt** | **568,000** |
| **Kapselhülle** | |
| Gelatine (Schwein), Glycerin, 99,5%, Titandioxid (E171), Eisenoxid, rot und schwarz (E172), Wasser, ger. | 245,000 |
| **Summe Kapselhülle** | **245,000** |
| **Gesamt: Kapsel** | **813,00** |

Die Wirkung der erfindungsgemäßen Zusammensetzung auf die Trainierbarkeit und die motorische und kognitive Leistungsfähigkeit nach körperlicher Erschöpfung wurde in einer placebokontrollierten Doppelblindstudie überprüft. Dabei erhielt die Verumgruppe zweimal täglich zwei Kapseln gemäß Beispielrezeptur, die Placebogruppe ein gleich aussehendes Placebo ohne Wirkstoff. Die 33 Versuchspersonen waren durchschnittlich ausdauertrainierte Studenten im Alter von 20 bis 35 Jahren, die vor der Einnahme des Präparats zahlreichen Untersuchungen unterzogen wurden. Danach erfolgte ein vierwöchiges kontrolliertes Ausdauertraining, und anschließend wurde der Anfangstest wiederholt. Im Mittelpunkt der Tests stand eine einstündige Dauerbelastung auf dem Fahrradergometer bei 70% der maximalen Sauerstoffaufnahme. Anschließend wurde ein erschöpfender Stufentest durchgeführt. Zur Beurteilung der Leistungsverbesserung durch das pulsgesteuerte Training dienen die im Stufentest nach einstündiger Dauerbelastung erreichte maximale Wattstufe und die Durchhaltezeit sowie die sich daraus ergebende Gesamtarbeit (Gesamtarbeit [Wmin] = Wattstufe [W] x Durchhaltezeit [min]).

Als Ergebnis war festzuhalten, dass sich beide Gruppen durch das vierwöchige intensive Ausdauertraining verbessert haben. In der Verumgruppe war diese Verbesserung jedoch deutlich größer. So verbesserte sich die Gesamtarbeit in der Placebogruppe um 317 Wmin, in der Verumgruppe um 660 Wmin. Die Steigerung in der Verumgruppe lag somit um 52% höher. Gleiches zeigte sich im Stufentest. Hier verbesserte sich die Placebogruppe um 512 Wmin, die Verumgruppe um 647 Wmin (+21%). Die deutliche Leistungssteigerung durch das Präparat zeigt sich auch in der maximal erreichten Wattstufe. Hier kam es in der Placebogruppe zu einer Entwicklung von 254 W auf 294 W, während die Verumgruppe sich von 250 W auf 315 W steigerte. Dass es sich um eine echte Leistungssteigerung handelt, zeigt die Entwicklung der Laktatspiegel. Die Verumgruppe hat die höheren Leistungen nicht dadurch erbracht, dass eine höhere Sauerstoffschuld eingegangen wurde. Die für die Verbrennung erforderliche aufgenommene Sauerstoffmenge, gemessen an der maximalen Sauerstoffaufnahme im Stufentest, erhöhte sich in der Verum-Gruppe nur leicht, während die Werte in der Placebogruppe deutlich anstiegen. Die höhere Leistung in der Verum-Gruppe wurde also mit deutlich weniger Anstrengung erbracht. Das erfindungsgemäße Präparat verbessert die Regenerationsfähigkeit nach körperlicher Belastung. Eine verbesserte Regenerationsfähigkeit ermöglicht gleichzeitig eine bessere Trainierbarkeit von Ausdauerleistungen.

Das Erholungsverhalten zwischen den beiden Gruppen war signifikant unterschiedlich. In der Verumgruppe war eine größere Erhaltung der Aufmerksamkeit und der zentralnervösen Aktivierung zu verzeichnen. Anhand von motorischen und kognitiven Tests konnten Ermüdungsreaktionen durch die erschöpfenden Belastungen aufgezeigt werden. Unter Verum-Einfluss war die Theta-Frequenz (Aufmerksamkeitssteuerung) erkennbar günstiger. Zahlreiche Gruppenunterschiede im EEG-Muster waren in der Erholungsphase nachweisbar. Es kam zu einer Ökonomisierung der corticalen Abläufe. Dies deckt sich mit Ergebnissen von Studien, die nachweisen, dass "Könner" bzw. "Experten" gleiche Aufgaben mit geringeren corticalen Ressourcen lösen.

Das erfindungsgemäße Präparat steigert also auch die kognitive Leistungsfähigkeit nach körperlicher Belastung.

## Patentansprüche

1. Pharmazeutische, medizinische, diätetische oder alimentäre Zusammensetzung, deren Wirkstoffzusammensetzung auf einer Kombination aus
Rhodiola-Extrakt, Astaxanthin und einem Antioxidans, ausgewählt aus der Gruppe Vitamin A, Vitamin C und Vitamin E
basiert.

2. Zusammensetzung nach Anspruch 1, **dadurch gekennzeichnet, dass** sie als Wirkstoff des Weiteren mindestens einen Mineralstoff, ausgewählt aus der Gruppe Calcium, Kalium, Magnesium, Natrium, Chlor, Eisen, Selen und Zink, enthält.

3. Zusammensetzung nach Anspruch 1 oder 2, **dadurch gekennzeichnet, dass** der Rhodiola-Extrakt ein wässrig-ethanolischer Rhodiola rosea Extrakt ist.

4. Zusammensetzung nach einem der Ansprüche 1 bis 3, **dadurch gekennzeichnet, dass** das Antioxidans ein Vitamin E ist.

5. Zusammensetzung nach einem der Ansprüche 1 bis 4, **dadurch gekennzeichnet, dass** die Wirkstoffzusammensetzung aus folgenden Bestandteilen besteht:
zwischen 10 und 75 Gew.-%, vorzugsweise zwischen 20 und 45 Gew.-%,
besonders bevorzugt zwischen 30 und 45 Gew.-% Rhodiola-Extrakt,
zwischen 2 und 30 Gew.-%, vorzugsweise zwischen 6 und 25 Gew.-%, besonders bevorzugt zwischen 9 und 21 Gew.-% Antioxidans,
zwischen 0,1 und 15 Gew.-%, vorzugsweise zwischen 0,2 und 6 Gew.-%,
besonders bevorzugt zwischen 0,3 und 3 Gew.-% Astaxanthin, und
zwischen 0 und 87,9 Gew.-%, vorzugsweise zwischen 25 und 75 Gew.-%, besonders bevorzugt zwischen 31 und 60,7 Gew.-% mindestens eines Mineralstoffes,
ausgewählt aus der Gruppe Calcium, Kalium, Magnesium, Natrium, Chlor, Eisen,
Selen und Zink.

6. Zusammensetzung nach einem der Ansprüche 1 bis 4, **dadurch gekennzeichnet, dass** sie des Weiteren Lecithin, L-Carnitin, ein oder mehrere Vitamine des B-Komplexes, eine oder mehrere essenzielle Aminosäuren und/oder eine oder mehrere essenzielle Fettsäuren enthält.

7. Zusammensetzung nach Anspruch 6, **dadurch gekennzeichnet, dass** die Wirkstoffzusammensetzung aus folgenden Bestandteilen besteht:
zwischen 10 und 75 Gew.-%, vorzugsweise zwischen 20 und 45 Gew.-%,
besonders bevorzugt zwischen 30 und 45 Gew.-% Rhodiola-Extrakt,
zwischen 2 und 30 Gew.-%, vorzugsweise zwischen 6 und 25 Gew.-%, besonders bevorzugt zwischen 9 und 21 Gew.-% Antioxidans,
zwischen 0,1 und 15 Gew.-%, vorzugsweise zwischen 0,2 und 6 Gew.-%,
besonders bevorzugt zwischen 0,3 und 3 Gew.-% Astaxanthin,
zwischen 5 und 90 Gew.-%, vorzugsweise zwischen 10 und 75 Gew.-%, besonders bevorzugt zwischen 25 und 45 Gew.-% mindestens einer Verbindung ausgewählt aus der Gruppe Lecithin, L-Carnitin, Vitamine des B-Komplexes, essenzielle Aminosäuren und essenzielle Fettsäuren und
zwischen 5 und 90 Gew.-%, vorzugsweise zwischen 10 und 75 Gew.-%, besonders bevorzugt zwischen 25 und 45 Gew.-% mindestens eines Mineralstoffes, ausgewählt aus der Gruppe Calcium, Kalium, Magnesium, Natrium, Chlor, Eisen, Selen und Zink.

8. Zusammensetzung nach einem der Ansprüche 1 bis 7, **dadurch gekennzeichnet, dass** sie als Hilfsstoff_mindestens ein Pflanzenöl enthält.

9. Zusammensetzung nach einem der Ansprüche 1 bis 8, **dadurch gekennzeichnet, dass** sie als Hilfsstoff mindestens eine Verbindung ausgewählt aus der Gruppe Siliziumdioxid, Polyvinylpyrrolidon, Maltodextrin, Magnesiumstearat, Cellulose und Carboxymethylstärke enthält.

10. Verwendung der Zusammensetzung nach einem der Ansprüche 1 bis 9 als diätetisches Lebensmittel.

11. Verwendung nach Anspruch 10 zur Verbesserung der Regeneration zwischen körperlichen Belastungen.

12. Verwendung nach Anspruch 10 oder 11 zur Verbesserung der kognitiven Leistungsfähigkeit nach körperlicher Belastung.
